# EUROPEAN PATENT SPECIFICATION

(11) **EP 1 557 128 B1**
(45) Date of publication and mention of the grant of the patent: **07.03.2007**
(21) Application number: 05000132.0
(22) Date of filing: 05.01.2005
(51) Int. Cl.: A61B 17/22, A61B 17/32

(54) **Atherectomy head and atherectomy catheter using the same**
Atherektomievorrichtung
Dispositif d'athérectomie

(30) Priority: 23.01.2004 JP 2004015503
(43) Date of publication of application: 27.07.2005
(73) Proprietor: Cathenet Corporation, Osaka 565-0853 (JP)
(72) Inventor: Kono, Hiroshi, Kobe-City Hyogo 655-0851 (JP)
(74) Representative: Banzer, Hans-Jörg

(56) References cited:
- US-A- 5 746 758
- US-B1- 6 235 042

## Description

### BACKGROUND OF THE INVENTION

### 1. Field of the Invention

The present invention relates to an atherectomy head for eliminating and removing a thrombus deposited in a blood vessel, and to an atherectomy catheter having the head connected to a tip thereof.

### 2. Description of the Related Art

In order to remove a thrombus deposited in a blood vessel (hereinafter simply "vessel"), a guide wire is inserted into the vessel through tissue of a patient and advanced along the vessel to a position of the thrombus to be removed, and after a catheter is reached to the thrombus, the guide wire is removed from the vessel. Subsequently, the thrombus is broken or dissolved by use of the catheter left in the vessel. Thereafter, the broken or dissolved thrombus is aspirated into the catheter. Thus, the thrombus is removed from a body of the patient. As a method for partially or entirely dissolving the thrombus, a thrombolytic agent such as streptokinase is injected into an area of the thrombus from a tip of the catheter. This method uses a large amount of the thrombolytic agent in expectation that the thrombolytic agent is diluted with blood. Accordingly, this method may cause bleeding, and adversely affects the other organs.

Besides such a method for breaking or dissolving the thrombus, there is a method of expanding a balloon provided on the tip of the catheter to expand the vessel, thereby restoring a blood flow (PTCA method). However, according to this method, more pressure than necessary is applied to an inner wall of the vessel over an entire length of the balloon, and a damage of the vessel is caused, which may trigger a further formation of the thrombus. In order to prevent such restenosis, a stent is indwelled in the vessel. However, such indwelling of the stent for a long time may bring a risk of causing the thrombus again.

As the method for breaking the thrombus, various methods have been proposed, such as a method of providing a spray nozzle on the tip of the catheter to spray water therefrom to break the thrombus, a method of breaking the thrombus by a laser beam, and a method of repeatedly colliding, with the thrombus, a solid composed of thin lines expandable into an umbrella shape from the tip of the catheter to break the thrombus. However, each of the above-mentioned methods has merits and demerits, and a satisfactory measure to break the thrombus has not been obtained yet. Besides such thrombus breaking measures as proposed above, an atherectomy catheter (trade name: Rotablader, made by Boston Scientific Corporation) to be described below is commercially available. In this atherectomy catheter, a head having a cutter composed of a file-shaped stainless steel body embedding diamond powder therein is attached onto a tip of the wire, and the head is rotated at extremely high speed. Thus, the atherectomy catheter pulverizes the thrombus into particles not larger than erythrocytes, absorbs the particles into phagocytes, and discharges the particles to an outside of the body through the liver.

In this atherectomy catheter, the head having the cutter, which is attached onto the tip of the wire, collides with the thrombus while the file-shaped stainless steel body embedding the diamond powder therein is rotating at the high speed, thereby eliminating the thrombus. Accordingly, there is a risk that the head collides not only with the thrombus but also with the inner wall of the vessel to damage the vessel, and a highly skilled treatment technique is required.

As means for solving such problems, atherectomy catheters which remove the thrombus without damaging the vessel by attaching the following heads onto the tips of the wires are introduced. The heads are: one which includes a semicircular blade made of an elastic material on a surface of an elimination member embedding the diamond powder in an elastic plastic material or stainless steel (refer to JP 07-79985 A); and one which includes projected lines composed of an elastic material on a surface of a semicircular plastic member embedding the diamond powder therein at equal intervals (refer to JP 07-108044 A).

In these atherectomy catheters, the blade and the projected lines which are made of the elastic materials are provided on the surfaces of the file-shaped elimination members in the heads attached onto the tips of the wires. Accordingly, as compared with the ablation member composed only of the file-shaped member embedding the diamond powder therein, the risk of damaging the inner wall of the vessel is lowered in these atherectomy catheters. However, the blade and the projected lines which are made of the elastic materials are easily abraded. Further, each surface of the file-shaped elimination members, which eliminates the thrombus, faces upward from a micro viewpoint, and accordingly, these atherectomy catheters have a drawback of damaging the inner wall of the vessel in the case of moving in the vessel while the elimination members are rotating. Moreover, when the thrombus is to be removed by use of these catheters in the case where the thrombus is formed only on one surface of the inner wall of the vessel, these catheters have a risk of damaging the other surface of the inner wall of the vessel where the thrombus is not formed because the catheters are advanced in the vessel in a state where the same counter pressure is applied thereby.

US6235042 discloses a rotary atherectomy head according to the preamble of claim 1.

### SUMMARY OF THE INVENTION

The present invention has been made to solve the above-mentioned problems inherent in the conventional atherectomy catheters. Therefore, the present invention has an object to provide an atherectomy head capable of eliminating the thrombus with the number of revolutions, which is smaller than the number of revolutions (200,000 rpm) of the head of the commercially available Rotablader, without damaging the inner wall of the vessel, and also to provide an atherectomy catheter having the head connected to a tip thereof.

According to the present invention, this object is achieved by an atherectomy head as defined by independent claim 1. The dependent claims define preferred and advantageous embodiments of the invention.

In order to achieve the above object, according to an aspect of the present invention, there is provided an atherectomy head, including: a blade for eliminating a thrombus; a blade member including the blade; and an elastic member formed of a material that is deformed by collision with a thrombus, the elastic member including an outer surface which is substantially the same as a surface of the blade or protrudes outwardly beyond the surface of the blade, in which the tip surface of the elastic member is dented backward from the surface of the blade by the collision with the thrombus, and the blade of the blade member abuts on the thrombus present in a dent formed to eliminate the thrombus.

Further, in the atherectomy head according to the aspect of the present invention, a groove for aspirating the eliminated thrombus into an inside of a catheter is formed.

Further, in the atherectomy head according to the aspect of the present invention, the outer surface of the elastic member is coated with a coating member made of a material that is not deformed by the collision with the thrombus, and the blade is in contact with the coating member. The blade can be prevented from biting the elastic member, and the elastic member can be prevented from being abraded by the thrombus subjected to calcification.

Further, in the atherectomy head according to the aspect of the present invention, at least two blades are provided each as the blade.

Further, in the atherectomy head according to the aspect of the present invention, the elastic member is a resilient springboard-like elastic member.

According to another aspect of the present invention, there is provided an atherectomy catheter, including: a cylindrical catheter body; a freely rotatable wire inserted into an inside of the catheter body; aspirating means for aspirating an eliminated thrombus into the inside of the catheter body; and the atherectomy head described above, which is provided on a tip of the atherectomy catheter.

Further, in the atherectomy catheter according to the aspect of the present invention, the atherectomy catheter is composed such that a visor-like member is provided on an exposed tip of the catheter body, and that the atherectomy head is provided on the tip of the catheter. By providing the visor-like member on the exposed tip of the catheter body, when the thrombus adhered onto only one inner wall of the vessel is eliminated, the other inner wall of the vessel can be prevented by the visor-like member from being damaged by the blade of the head.

According to another aspect of the present invention, there is provided an atherectomy catheter, including: a freely rotatable hollow cylindrical wire; rotating means for rotating the cylindrical wire, the rotating means being provided on a rear end thereof; aspirating means for aspirating an eliminated thrombus into an inside of the cylindrical wire, the aspirating means being provided on a rear end thereof; and the atherectomy head described above, which is provided on a tip of the cylindrical wire.

According to another aspect of the present invention, there is provided an atherectomy catheter, comprising: a freely rotatable cylindrical catheter body containing fiber; rotating means for rotating the catheter body, the rotating means being provided on a rear end thereof; aspirating means for aspirating an eliminated thrombus into an inside of the catheter body, the aspirating means being provided on a rear end thereof; and the atherectomy head described above, which is provided on a tip of the catheter body.

According to another aspect of the present invention, there is provided an atherectomy catheter, comprising: a freely rotatable cylindrical catheter body containing a mesh; rotating means for rotating the catheter body, the rotating means being provided on a rear end thereof; aspirating means for aspirating an eliminated thrombus into an inside of the catheter body, the aspirating means being provided on a rear end thereof; and the head described above, which is provided on a tip of the catheter body.

As described above, in the head of the present invention, the elastic member provided together with the blade member for eliminating the thrombus collides with the thrombus. Thus, the elastic member is dented, and the outer surface of the elastic member is slightly dented backward from the surface of the blade of the blade member. By use of a step difference thus formed, the blade of the blade member abuts on the thrombus, and the thrombus is eliminated. Meanwhile, even if the tip of the head collides with softer one than the elastic member, such as the inner wall of the vessel, the elastic member is not deformed. Accordingly, even if the head rotates, the blade of the blade member does not bite the wall of the vessel. Moreover, the blade eliminates the thrombus inward. Accordingly, even if the head is one in which a gap is formed between the blade and the elastic member, the elastic member performs a protection function for the wall of the vessel, and the blade does not bite the wall of the vessel. As a result, the atherectomy catheter of the present invention can eliminate the thrombus deposited in the vessel without damaging the wall of the vessel. Heretofore, when the thrombus is attempted to be removed by inserting the catheter into the vessel in which the thrombus is adhered onto only one inner wall, the other inner wall onto which the thrombus is not adhered may be liable to damage. However, such a problem is solved by providing the visor-like member on the exposed tip of the catheter body.

### BRIEF DESCRIPTION OF THE DRAWINGS

FIG. 1 is a partially cutaway side view of an atherectomy catheter of the present invention.
FIG. 2 is a partially cutaway side view showing another example of the atherectomy catheter of the present invention.
FIG. 3-1 is a schematic side view showing another example of the atherectomy catheter of the present invention.
FIG. 3-2 is a schematic plan view of FIG. 3-1.
FIG. 4 is a partially cutaway schematic view showing another example of the atherectomy catheter of the present invention.
FIG. 5 is a partially cutaway schematic view showing another example of the atherectomy catheter of the present invention.
FIG. 6 is a partially cutaway side view showing another example of the atherectomy catheter of the present invention.
FIG. 7 is a side view of an atherectomy catheter in which a guide wire is inserted into a hollow portion of the hollow drive wire of FIG.6.
FIG. 8 is a plan view of the head of FIG. 1, viewed from a bottom thereof.
FIG. 9 is a side view of the head viewed from an opposite side of the side view of the head of FIG. 1.
FIG. 10 is a plan view of the tip bearing of FIG. 1, viewed from a tip thereof.
FIG. 11 is a side view of a head in which a coating member is provided on a tip surface of an elastic member.
FIG. 12 is a side view of the head viewed from an opposite side of the side view of the head of FIG. 11.
FIG. 13 is a plan view of the head viewed from a bottom thereof.
FIG. 14 is a plan view of a head viewed from a bottom thereof, showing another example different from that of FIG. 13.
FIG. 15 is a plan view of a head viewed from a bottom thereof, showing another example different from that of FIG. 14.
FIG. 16 is a side view of a head, in which a gap is provided between a surface of a blade and a tip surface of an elastic member, and the surface of the blade and the tip surface of the elastic member are formed at substantially the same height.
FIG. 17 is a side view of the head viewed from an opposite side of the side view of the head of FIG. 16.
FIG. 18 is a plan view of the head of FIG. 16, viewed from a bottom thereof.
FIG. 19 is a plan view of a head viewed from a bottom thereof, in which a pair of blades and a pair of elastic members are placed at positions substantially orthogonal to each other, and spaces are formed between a blade member and the elastic members, which are adjacent to each other.
FIG. 20 is a side view of the head of FIG. 19.
FIG. 21 is a plan view of a head having eight blades, viewed from a bottom thereof.
FIG. 22 is a plan view of a head in which a spring board is used, viewed from a bottom thereof.
FIG. 23 is a side view of the head of FIG. 22.
FIG. 24 is a plan view of a head of another example different from that of FIG. 22, viewed from a bottom thereof.

### DETAILED DESCRIPTION OF THE PREFERRED EMBODIMENTS

Embodiments of the present invention will be described below based on the drawings.

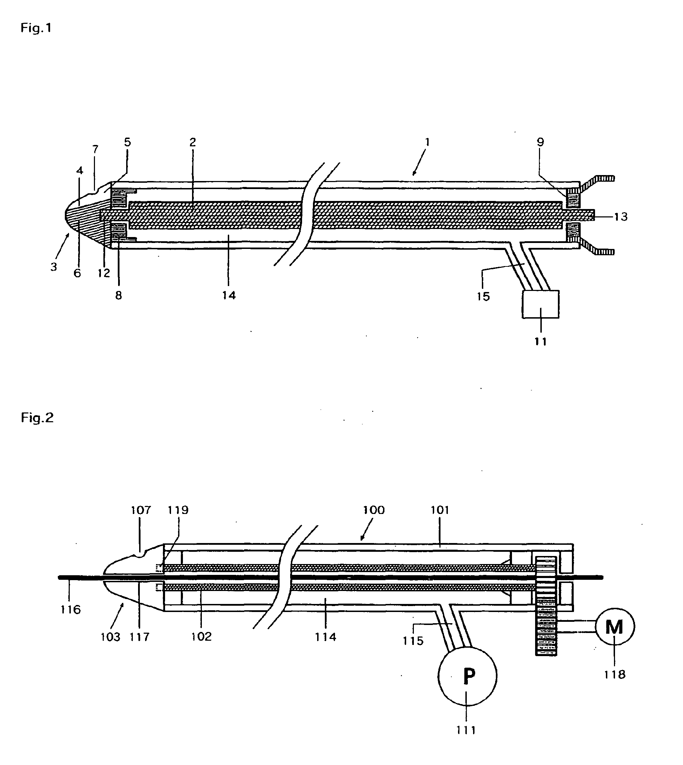

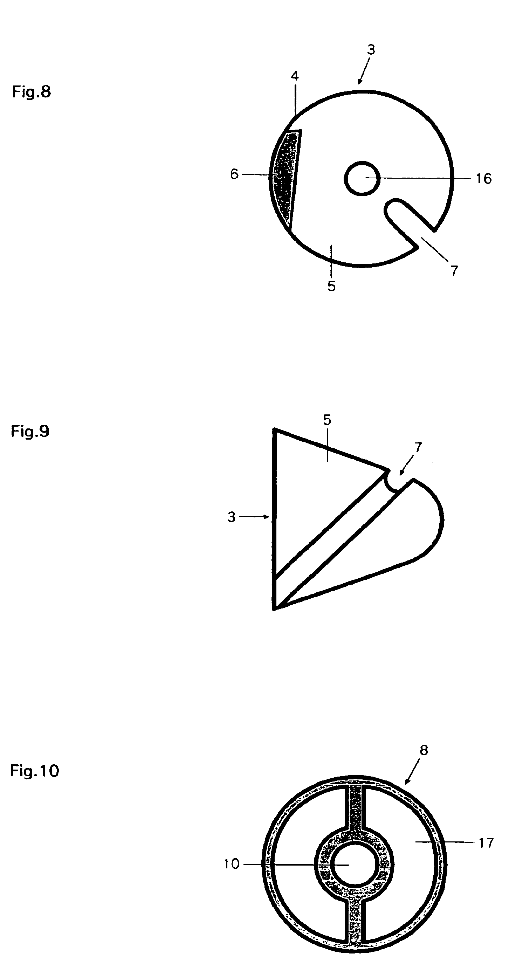

FIG. 1 is a partially cutaway side view of an atherectomy catheter of the present invention. The atherectomy catheter of the present invention includes a cylindrical catheter body 1, a freely rotatable drive wire 2 inserted into an inside 14 of the catheter body 1, a head 3 for eliminating a thrombus, which is provided on a tip of the catheter body 1, and aspirating means 11 for aspirating the eliminated thrombus into the inside 14 of the catheter body 1. The head 3 is composed of a blade member 5 having a blade 4 for eliminating the thrombus, and an elastic member 6 made of a material deformed by collision with the thrombus. An outer surface of the elastic member 6 is dented by the collision with the thrombus and moves backward from the blade 4 of the blade member 5, and the blade 4 of the head eliminates the thrombus present in the dent. On a rear end side of the catheter body 1, the aspirating means 11 such as a vacuum device and a syringe is placed. The aspirating means 11 turns the inside 14 of the catheter body 1 to a negative pressure state, and the eliminated thrombus is aspirated from a groove 7 formed on an outer surface of the head through a hole 17 of a tip bearing 8, which is shown in FIG. 10, into the inside 14 of the catheter body 1, and then aspirated from a passage 15 into the aspirating means 11. What is given as the elastic material is nonrigid plastic, a rubber-like substance and the like, such as polyvinyl chloride, polyurethane, and nylon.

The catheter body 1 is a hollow cylindrical tube which is formed of a flexible material such as polyvinyl chloride, polyurethane, nylon and Teflon (registered trademark), with both ends open. The catheter body 1 is capable of advancing or reversing in an inside of a vessel so as to be freely slidable. The drive wire 2 is formed of stainless steel such as SUS304, and is inserted into the inside 14 of the catheter body 1. A rear end 13 of the drive wire 2 protrudes from a rear end bearing 9 provided on a rear end of the catheter body 1 to be connected to a motor, making the drive wire 2 rotatable. Moreover, a tip 12 of the drive wire 2 protrudes forward from the tip bearing 8 provided on the tip of the catheter body 1. The tip 12 is connected by adhesive and the like to a hole 16 formed in the head 3, which is shown in FIG. 8, through a hole portion 10 of the tip bearing 8, which is shown in FIG. 10. The connection of the drive wire 2 and the head 3 may also be made by screwing, engaging and welding. Moreover, a discharge of a thrombus waste can be prompted by providing a helical rib on an outer surface of the drive wire 2.

FIG. 8 is a plan view of the head of FIG. 1, viewed from a bottom thereof, and FIG. 9 is a side view of the head viewed from an opposite side of the side view of the head of FIG. 1. The head 3 is formed of the hard blade member 5 and the elastic member 6. The blade member 5 has the sharp blade 4 for eliminating the thrombus and is composed of titanium, stainless steel or the like. The elastic member 6 is composed of a material deformed into a concave shape by the collision with the thrombus, that is, a softer material than the thrombus, for example, polyurethane, vinyl chloride, nylon, silicone or the like. On the head 3, the groove 7 for aspirating the eliminated thrombus into the inside 14 of the catheter body 1 is formed. It is preferable that the groove 7 be formed in the blade member 5. It is preferable that the head 3 have a conic shape, a hemispherical shape and the like. The head 3 is formed in such a manner that the blade member 5 having the blade 4 and the elastic member 6 are brought into contact with each other. The tip surface of the elastic member 6 is in contact with the blade member 5 so as to be placed on substantially the same surface as a tip surface of the blade member 5, that is, a surface of the blade 4, or to protrude forward therefrom. The elastic member 6 also includes a coating member coated on the tip of the elastic member 6. Contact surfaces of the blade member 5 and the elastic member 6 other than the vicinity of the surface of the blade 4 are joined together by the adhesive and the like.

The elastic member 6 collides with the thrombus, and thus the elastic member 6 is dented. The outer surface of the elastic member 6 is placed backward from the surface of the blade 4 of the blade member 5, and the blade 4 of the blade member 5 of the rotating head 3 abuts slightly on the thrombus present in the concave, thereby eliminating the thrombus. The eliminated thrombus is aspirated from the groove 7 formed on the head 3 through the hole 17 of the tip bearing 8, which is shown in FIG. 10, into the inside 14 of the catheter body 1. The tip surface of the elastic member 6 and the tip surface of the blade member 5, that is, the surface of the blade 4 are in contact with each other on substantially the same surface. As shown in FIG. 9, the groove 7 is provided on an outer surface of the blade member 5.

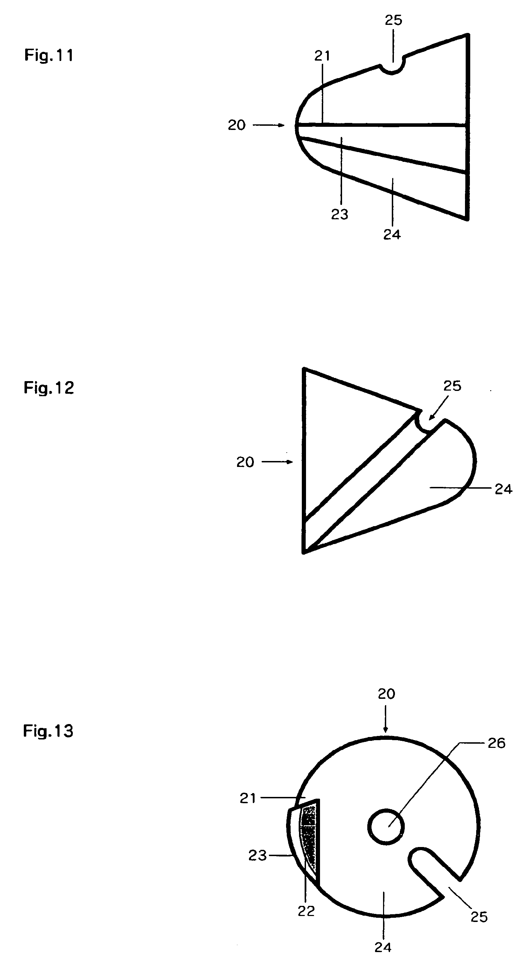

FIG. 11 is a side view of a head in which a coating member is provided on an outer surface of an elastic member, which is a side view of a head in which an outer surface of the coating member provided on the outer surface of the elastic member is formed more forward than a surface of a blade. FIG. 12 is a side view of the head viewed from an opposite side of the side view of the head of FIG. 11. FIG. 13 is a plan view of the head viewed from a bottom thereof. A head 20 is composed by being coated with a coating member 23 in which an outer surface of an elastic member 22 is formed of a material undeformed by the collision with the thrombus. As a material of the coating member 23, metal such as titanium and stainless steel, plastic such as Teflon (registered trademark), and the like are preferable.

An outer surface of the coating member 23 provided on the tip surface of the elastic member 22 is formed at a position protruding forward from a surface of a blade 21, and the surface of the blade 21 is formed in contact with the coating member 23. When the coating member 23 collides with the thrombus, the elastic member 22 is dented, the outer surface of the coating member 23 is dented more than the surface of the blade 21, and the blade 21 of a blade member 24 of the rotating head 20 eliminates the thrombus present in the dent, that is, a gap between the outer surface of the coating member 23 and the blade 21. The coating member 23 is provided on the elastic member 22 by the adhesive and the like, thus making it possible to prevent a risk that the blade 21 of the blade member 24 bites the elastic member 22. The eliminated thrombus is aspirated from a groove 25 provided on an outer surface of the blade member 24 into the inside 14 of the catheter body 1. Reference numeral 26 of a center of the head 20 of FIG. 13 denotes a hole of the head 20, which is adhered to the tip 12 of the drive wire 2. The head 20 is formed in such a manner that the coating member 23 provided on the outer surface of the elastic member 22 by the adhesive and the like is brought into contact with the surface of the blade 21, and the groove 25 is formed on the blade member 24. Contact surfaces of the blade member 24 and the elastic member 22 other than the vicinity of the surface of the blade 21 are joined together by the adhesive and the like.

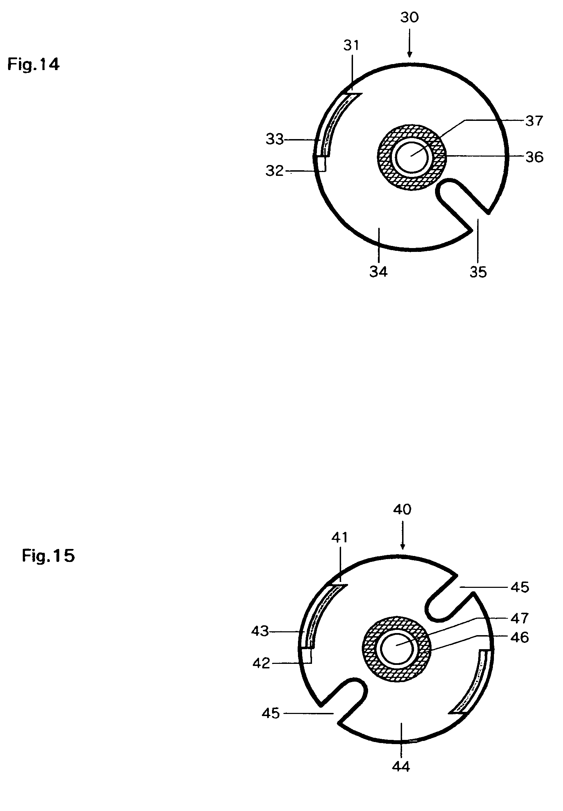

FIG. 14 is a plan view of a head viewed from a bottom thereof, showing another example different from that of FIG. 13. FIG. 14 shows a head 30 in which an outer surface of a coating member provided on an outer of an elastic member is formed at substantially the same position as a surface of a blade. In the head 30, a tip surface of a coating member 33 provided on a tip surface of an elastic member 32 is formed at a position of which height is substantially the same as that of a surface of a blade 31, and the surface of the blade 31 is formed in contact with the coating member 33. The eliminated thrombus is aspirated from a groove 35 provided on an outer surface of a blade member 34 into the inside of the catheter body. Reference numeral 37 of a center of the head 30 denotes a head passage 117 into which a guide wire 116 of an atherectomy catheter 100 of FIG. 2 is inserted, and reference numeral 36 denotes a hole for joining a head 103 and tip 119 of a hollow drive wire 102 of the atherectomy catheter 100 of FIG. 2 to each other by the adhesive and the like.

FIG. 15 is a plan view of a head viewed from a bottom thereof, showing another example different from that of FIG. 14. FIG.15 shows a head 40 in which an outer surface of a coating member provided on a tip of an elastic member is formed at substantially the same position as a surface of a blade and two grooves are provided. In the head 40, an outer surface of a coating member 43 provided on an outer surface of an elastic member 42 is formed at a position of which the height is substantially the same as that of a surface of a blade 41, and the surface of the blade 41 is formed in contact with the coating member 43. When the coating member 43 collides with the thrombus, the elastic member 42 is dented, the outer surface of the coating member 43 is dented more than the surface of the blade 41, and the blade 41 of the rotating head 40 eliminates the thrombus present in a gap between the outer surface of the coating member 43 and the surface of the blade 41. The eliminated thrombus is aspirated from two grooves 45 provided on an outer surface of a blade member 44 into the inside of the catheter body. Reference numeral 47 at a center of the head 40 denotes the head passage 117 into which the guide wire 116 of the atherectomy catheter 100 of FIG. 2 is inserted, and reference numeral 46 denotes a hole for joining the head 103 and tip 119 of the hollow drive wire 102 of the atherectomy catheter 100 of FIG. 2 to each other by the adhesive and the like.

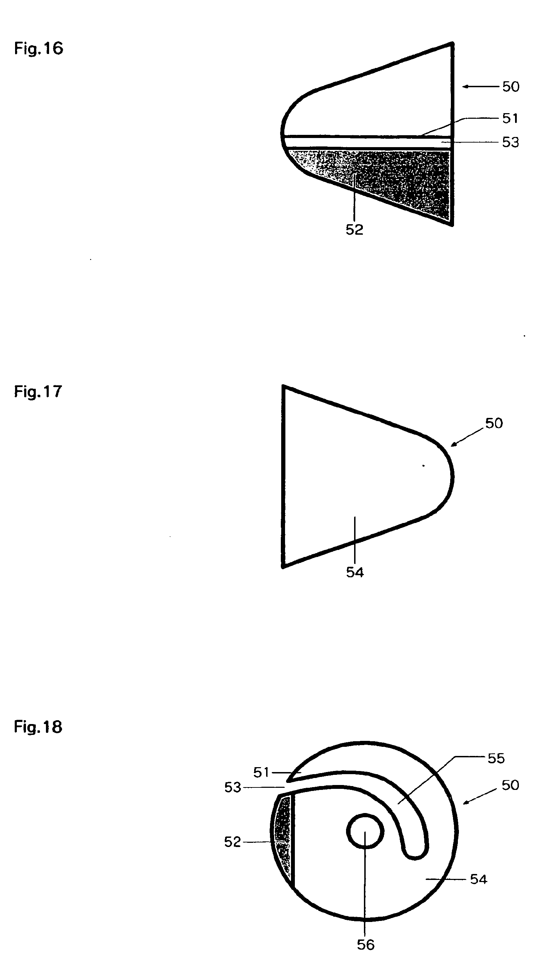

FIG. 16 is a side view of a head showing another example different from the above-described heads, in which a gap is provided between a surface of a blade and an outer surface of an elastic member, and the surface of the blade and the outer surface of the elastic member are formed at substantially the same height. FIG. 17 is a side view of the head viewed from an opposite side of the side view of the head of FIG. 16, and FIG. 18 is a plan view of the head of FIG. 16, viewed from a bottom thereof. In a head 50 of FIGS. 16 to 18, a surface of a blade 51 is formed in the same direction as a principal axis of the atherectomy catheter, and a gap 53 is formed between a tip surface of an elastic member 52 and the surface of the blade 51. Contact surfaces of the blade member 54 and the elastic member 52 other than the vicinity of the surface of the blade 51 are joined together by the adhesive and the like. The thrombus colliding with the head 50 is eliminated in an inward direction by the blade 51 of the rotating head 50. At this time, even if the head 50 collides with the inner wall of the vessel, the elastic member 52 is not dented because the inner wall of the vessel is softer than the elastic member 52. Then, the elastic member 52 performs a protection function for the inner wall of the vessel, and does not damage the inner wall of the vessel. A groove 55 is provided from the gap 53 through an inside of the blade member 54 to the bottom of the head, and the thrombus falling into the gap 53 is aspirated from the groove 55 into the inside of the catheter body.

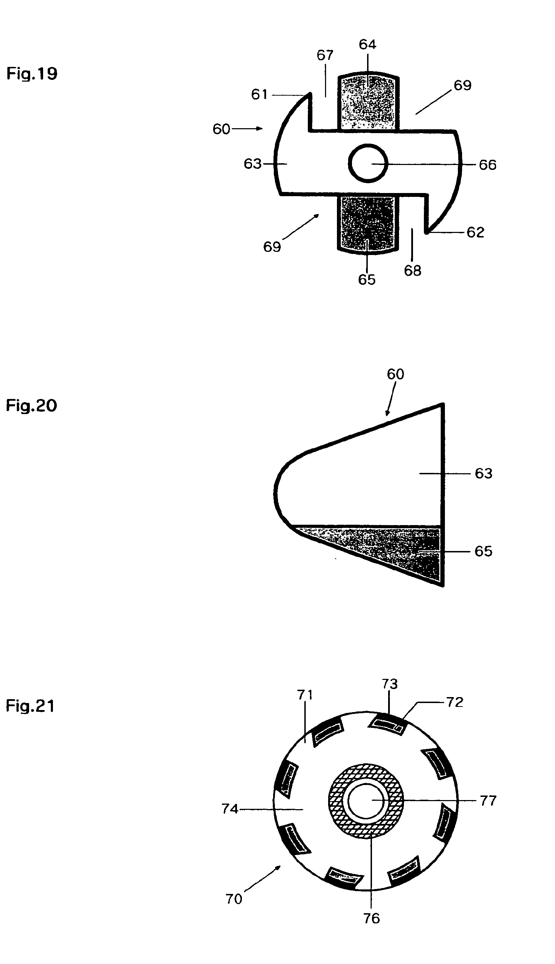

FIG. 19 is a plan view of a head viewed from a bottom thereof, in which a pair of blades and a pair of elastic members are placed at positions substantially orthogonal to each other, and spaces are formed between a blade member and the elastic members, which are adjacent to each other. FIG. 20 is a side view of the head of FIG. 19. A head 60 is composed of a blade member 63 in which a blade 61 and a blade 62 are placed outward opposite to each other, and an elastic member 64 and an elastic member 65, which are placed at positions substantially orthogonal to the blade member 63. The eliminated thrombus is aspirated into the inside of the catheter body from a space 67 formed between the blade 61 and the elastic member 64, a space 68 formed between the blade 62 and the elastic member 65, and spaces 69 formed between the blade member 63 and the elastic members 64 and 65.

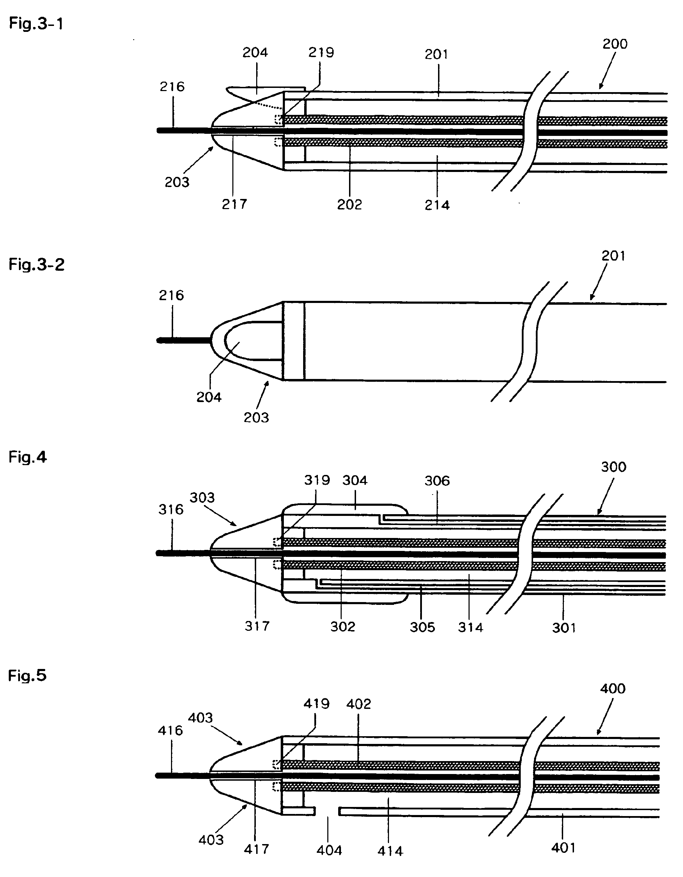

FIG. 21 is a plan view of a head having eight blades, viewed from a bottom thereof. A head 70 is formed in the following manner. Eight blades 71 and eight layered products of elastic members 72 and coating members 73 provided on outer surfaces thereof are arranged such that outer surfaces of the coating members 73 are placed at positions of which height is substantially the same as that of surfaces of the blades 71, and the surfaces of the blades 71 and the coating members 73 are brought into alternate contact with one another. When the coating members 73 collide with the thrombus, the elastic members 72 are dented, the outer surfaces of the coating members 73 are dented more than the surfaces of the blades 71, and the blades 71 of the rotating head 70 eliminate the thrombus present in the dents, that is, gaps between the outer surfaces of the coating members 73 and the surfaces of the blades 71. The head 70 is effective in the case of eliminating thrombus where calcification develops. However, in the head 70, no spot for providing a groove which guides the eliminated thrombus into the inside of the catheter body is provided in a blade member 74. Accordingly, unlike the heads of the other embodiments, no groove is formed in the head 70. Therefore, the eliminated thrombus floats in the vessel. Hence, by use of an atherectomy catheter 400 as shown in FIG. 5, in which a side hole 404 is provided on an outer circumference of a tip of a catheter body 401, the eliminated thrombus present in the vessel is aspirated from the side hole 404 into the inside of the catheter body. Reference numeral 77 at a center of the head 70 denotes a head passage 417 into which a guide wire 416 of the atherectomy catheter 400 of FIG. 5 is inserted. Reference numeral 76 denotes a hole for joining a head 403 and tip 419 of a hollow drive wire 402 of the atherectomy catheter 400 of FIG. 5 to each other by the adhesive and the like.

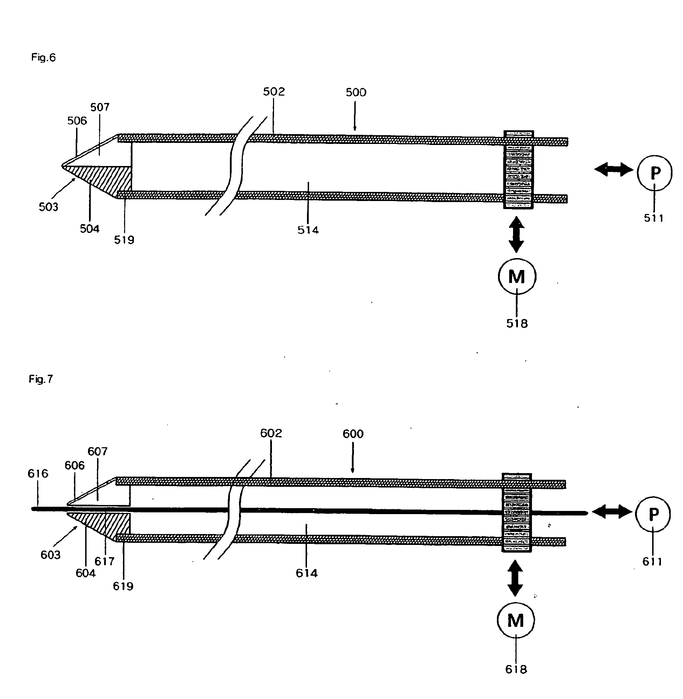

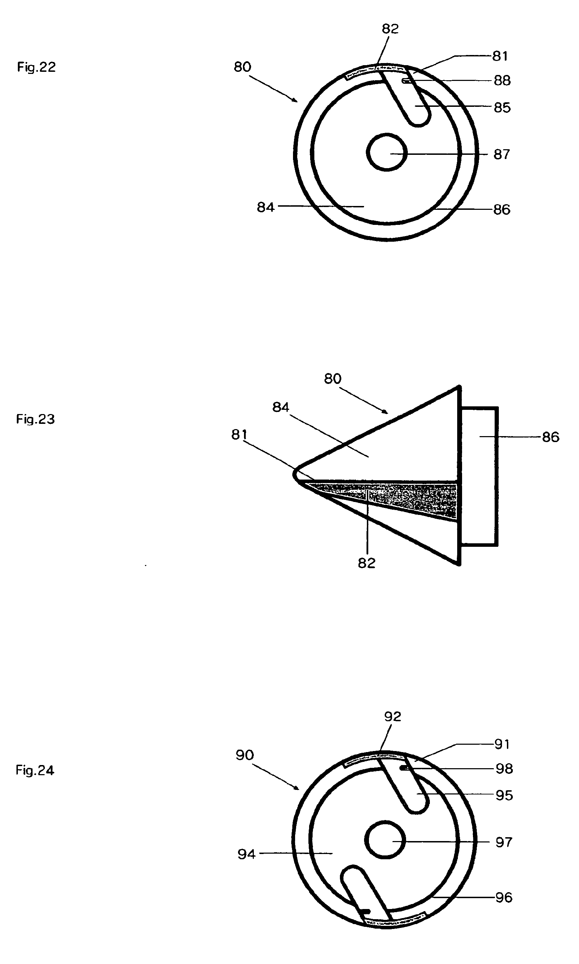

FIG. 22 is a plan view of a head in which a springboard-like elastic member is used, viewed from a bottom thereof, and FIG. 23 is a side view of the head. The head is one in which a resilient springboard-like elastic member is used as the elastic member instead of the elastic material such as the nonrigid plastic and the rubber-like substance. The head is a head 80 in which a surface of a blade and an outer surface of the springboard-like elastic member are formed to have substantially the same heights. The head 80 has a structure in which a spring board 82 is in contact with a tip of a surface side of a blade 81 and formed to cover a groove 85. When the springboard-like elastic member 82 collides with the thrombus, a tip of the springboard-like elastic member 82 is flexed more than a surface of the blade 81 and bent toward the groove 85 side, and the blade 81 of the rotating head 80 eliminates the thrombus present in a gap between the tip of the springboard-like elastic member 82 and the surface of the blade 81. A protrusion 88 provided in the groove 85 is one for preventing the tip of the spring board 82 from bending too much and preventing a risk that exposure of the surface of the blade 81 becomes large and that the surface of the blade 81 deeply bites the thrombus. The protrusion 88 serves as a stopper. The eliminated thrombus is aspirated from the groove 85 provided in a blade member 84 into the inside of the catheter body. The springboard-like elastic member is elastic metal which is a resilient thin plate. For the springboard-like elastic member, it is preferable to use stainless steel, a nickel-titanium alloy and the like. In FIG. 22, reference numeral 87 at a center of the head 80 denotes a head passage 617 into which a guide wire 616 of a hollow drive wire 602 of FIG. 7 is inserted. Reference numeral 86 of FIG. 22 denotes a convex portion for joining an inner wall of a tip 619 of the hollow drive wire 602 and a sidewall of a bottom of a head 603 of FIG. 7 to each other by the adhesive and the like.

FIG. 24 is a plan view of a head of another example different from that of FIG. 22, viewed from a bottom thereof. The head is a head 90 in which atherectomy portions are individually formed in two grooves 95. In each of the atherectomy portions, a surface of a blade and an outer surface of a springboard-like elastic member are formed to have substantially the same heights. In the grooves 95, protrusions 98 are individually formed. The head 90 has a structure in which springboard-like elastic members 92 are in contact with a tip of a surface side of a blade 91 and individually formed to cover the two grooves 95. When the springboard-like elastic members 92 collide with the thrombus, tips of the springboard-like elastic members 92 are flexed more than a surface of the blade 91 and bent toward the grooves 95 side, and the blade 91 of the rotating head 90 eliminates the thrombus present in gaps between the tips of the springboard-like elastic members 92 and the surface of the blade 91. The eliminated thrombus is aspirated from the grooves 95 provided on a blade member 94 into the inside of the catheter body. Reference numeral 97 at a center of the head 90 denotes the head passage 617 into which the guide wire 616 of the hollow drive wire 602 of FIG. 7 is inserted. Reference numeral 96 denotes a convex portion for joining the inner wall of the tip 619 of the hollow drive wire 602 and a sidewall of the bottom of the head 603 of FIG. 7 to each other by the adhesive and the like.

FIG. 2 is a partially cutaway side view showing another example of the atherectomy catheter of the present invention. The atherectomy catheter 100 includes a cylindrical catheter body 101, a freely rotatable hollow drive wire 102 inserted into an inside 114 of the catheter body 101, a head 103 for eliminating the thrombus, which is provided on a tip of the catheter body 101, and a vacuum pump 111 for aspirating the eliminated thrombus into the inside 114 of the catheter body 101. Into a hollow portion of the hollow drive wire 102, a guide wire 116 is inserted, and a tip thereof protrudes to the outside through a head passage 117 penetrating from a tip of the head 103 to a bottom thereof. Reference numeral 119 of the tip of the hollow drive wire 102 denotes a connecting portion of the hollow drive wire 102 and the head 103, which are connected to the head 103 by the adhesive and welding. The vacuum pump 111 placed on a rear end side of the catheter body 101 turns the inside 114 of the catheter body 101 to a negative pressure state, and the thrombus eliminated by the head 103 is aspirated from the inside 114 of the catheter body 101 through a passage 115 into the vacuum pump 111.

FIGS. 3-1 and 3-2 are partially cutaway views showing another example of the atherectomy catheter of the present invention: FIG. 3-1 is a schematic side view; and FIG. 3-2 is a schematic plan view. FIGS. 3-1 and 3-2 show an atherectomy catheter 200 characterized in that a visor-like member 204 is placed on an open tip of a catheter body 201. The atherectomy catheter 200 includes the cylindrical catheter body 201, a freely rotatable hollow drive wire 202 inserted into an inside 214 of the catheter body 201, a head 203 for eliminating the thrombus, which is provided on the tip of the catheter body 201, an aspiration device (not shown) for aspirating the eliminated thrombus into the inside 214 of the catheter body 201, and the visor-like member 204 placed on the open tip of the catheter body 201. Into a hollow portion of the hollow drive wire 202, a guide wire 216 is inserted, and a tip thereof protrudes to the outside through a head passage 217 penetrating from a tip of the head 203 to a bottom thereof. The visor-like member 204 is placed on the open tip of the catheter body 201. By providing the visor-like member 204 on the open tip of the catheter body 201, when the thrombus adhered onto only one inner wall of the vessel is eliminated, the other inner wall of the vessel is prevented by the visor-like member 204 from being damaged by a blade of the head 203. For the visor-like member 204, a thin plate made of metal, plastic or the like is used. Moreover, though the visor-like member 204 has a cap brim shape in FIG. 2, any shape can be adopted as long as the visor-like member 204 does not inhibit rotation of the head 203 and prevents the vessel from being damaged by the rotation thereof.

FIG. 4 is a partially cutaway schematic view showing another example of the atherectomy catheter of the present invention. FIG. 4 shows an atherectomy catheter 300 characterized in that a balloon 304 is placed on an outer circumference of a tip of a catheter body 301, and that a coolant passage 305 for a coolant for cooling heat generated by a hollow drive wire 302 rotating in the catheter body 301 and for washing an inside 314 of the catheter body 301 is placed. The atherectomy catheter 300 is composed of the cylindrical catheter body 301, a freely rotatable hollow drive wire 302 inserted into the inside 314 of the catheter body 301, a head 303 for eliminating the thrombus, which is provided on a tip of the catheter body 301, an aspiration device (not shown) for aspirating the eliminated thrombus into the inside 314 of the catheter body 301, the balloon 304 provided on the outer circumference of the tip of the catheter body 301, and the coolant passage 305 provided on an inner circumference of the catheter body 301. A guide wire 316 is inserted into a hollow portion of the hollow drive wire 302, and a tip thereof protrudes to the outside through a head passage 317 penetrating from a tip of the head 303 to a bottom thereof. The balloon 304 is expanded by a normal saline solution or the like which is supplied from a supply passage 306. The balloon 304 functions to maintain the vessel, to stabilize the catheter body 301 in the vessel for preventing a position of the head 303 from shaking, and to stop the flow of blood when it needed. Moreover, the coolant passage 305 is provided on the inner circumference of the catheter body 301, and cools the heat generated by the rotating hollow drive wire 302. Furthermore, the coolant supplied from the coolant passage 305 is aspirated through the inside 314 of the catheter body 301 into the vacuum device (not shown), and accordingly, functions to wash and eliminate the thrombus and the like which are aspirated into the inside 314 of the catheter body 301.

FIG. 5 is a partially cutaway schematic view showing another example of the atherectomy catheter of the present invention. An atherectomy catheter 400 shown in FIG.5 is optimum for the case of using the head 70 without any groove, which is as shown in FIG. 21, and is characterized in that a side hole 404 is provided on an outer circumference of a tip of a catheter body 401 to aspirate and remove the eliminated thrombus present in the vessel from the side hole 404. The atherectomy catheter 400 is composed of the cylindrical catheter body 401, a freely rotatable hollow drive wire 402 inserted into an inside 414 of the catheter body 401, a head 403 for eliminating the thrombus, which is provided on the tip of the catheter body 401, a vacuum device (not shown) for aspirating the eliminated thrombus into the inside 414 of the catheter body 401, and the side hole 404 provided on the outer circumference of the tip of the catheter body 401. A guide wire 416 is inserted into a hollow portion of the hollow drive wire 402, and a tip of the guide wire 416 protrudes to the outside through a head passage 417 penetrating from a tip of the head 403 to a bottom thereof. The head having eight blades, which is shown in the plan view of the head of FIG. 21, viewed from the bottom thereof, does not have a dead space for forming the grooves for removing the eliminated thrombus. Accordingly, the eliminated thrombus floats in the vessel. The thrombus concerned is aspirated from the side hole 404 provided on the outer circumference of the tip of the catheter body 401 into the inside 414, and is removed to the vacuum device (not shown).

FIG. 6 is a partially cutaway side view showing another example of the atherectomy catheter of the present invention. An atherectomy catheter 500 includes a freely rotatable hollow drive wire 502, a head 503 for eliminating the thrombus, which is provided on a tip of the hollow drive wire 502, a vacuum pump 511 for aspirating the eliminated thrombus into an inside 514 of the hollow drive wire 502, and a motor 518 for rotating the hollow drive wire 502. The head 503 has a structure in which a springboard-like elastic member 506 is in contact with a tip of a surface side of a blade (hidden and invisible in the drawing) and formed to cover a groove 507. When the springboard-like elastic member 506 collides with the thrombus, a tip of the springboard-like elastic member 506 is flexed more than the surface of the blade and bent toward the groove 507 side, and the blade of the rotating head 503 eliminates the thrombus present in a gap between the tip of the springboard-like elastic member 506 and the surface of the blade. The vacuum pump 511 placed on a rear end side of the hollow drive wire 502 turns the inside 514 of the hollow drive wire 502 to a negative pressure state, and the thrombus eliminated by the head 503 is aspirated from the groove 507 provided on a blade member 504 through the inside 514 of the hollow drive wire 502 into the vacuum pump 511.

FIG. 7 is a side view of an atherectomy catheter in which a guide wire 616 is inserted into a hollow portion of the hollow drive wire 502 of FIG.6. An atherectomy catheter 600 includes a freely rotatable hollow drive wire 602, a head 603 for eliminating the thrombus, which is provided on a tip of the hollow drive wire 602, a vacuum pump 611 for aspirating the eliminated thrombus into an inside 614 of the hollow drive wire 602, and a motor 618 for rotating the hollow drive wire 602. A guide wire 616 is inserted into the hollow portion of the hollow drive wire 602, and a tip thereof protrudes to the outside through a head passage 617 penetrating from a tip of the head 603 to a bottom thereof. The vacuum pump 611 placed on a rear end side of the hollow drive wire 602 turns the inside 614 of the hollow drive wire 602 to a negative pressure state, and the thrombus eliminated by the head 603 is aspirated from a groove 607 provided in a blade member 604 through the inside 614 of the hollow drive wire 602 into the vacuum pump 611. Depending on an occlusion state of the thrombus in the vessel, the guide wire 616 is replaced by a guide wire having a file attached onto the tip of the guide wire 616 concerned, and a tip of the guide wire is slightly protruded from the head 603 and rotated together with the hollow drive wire 602, thus making it possible to eliminate the thrombus subjected to calcification. Moreover, the head of the present invention can be used by also being attached onto a tip of a catheter containing a mesh or fiber, in which the cylindrical body is made of plastic containing the mesh or fiber, instead of the catheter in which the cylindrical body of the hollow drive wire is made of stainless steel.

## Claims

1. An atherectomy head, comprising:
a blade (4; 21; 31; 41; 51; 61; 71; 81; 91) for eliminating a thrombus;
a blade member (5; 24; 34; 44; 54; 63; 74; 84; 94) including the blade; and
an elastic member (6; 22; 32; 42; 52; 65; 72; 82; 92) formed of a material that is deformed by collision with a thrombus, the elastic member including an outer surface which comprises one of a surface at substantially the same height on the head as a surface of the blade; and **characterised in that** said surface protrudes outwardly beyond the surface of the blade,
such that the tip surface of the elastic member is dented backward from the surface of the blade by the collision with the thrombus, and the blade of the blade member abuts on the thrombus present in a dent formed to eliminate the thrombus.

2. An atherectomy head according to claim 1, wherein a groove (7; 25; 35; 45; 55; 85; 95) for aspirating the eliminated thrombus into an inside of a catheter ((1; 101; 201; 301; 401) is formed.

3. An atherectomy head according to claim 1 or 2, wherein:
the outer surface of the elastic member (22; 32; 42; 72) is coated with a coating member (23; 33; 43; 73) made of a material that is not deformed by the collision with the thrombus; and
the blade (21; 31; 41; 71) is in contact with the coating member.

4. An atherectomy head according to any one of claims 1 to 3, wherein at least two blades (61; 71) are provided each as the blade.

5. An atherectomy head according to any one of claims 1 to 4, wherein the elastic member comprises a resilient springboard-like elastic member (82; 92).

6. An atherectomy catheter, comprising:
a cylindrical catheter body (1; 101; 201; 301; 401);
a freely rotatable wire (2; 102; 202; 302; 402; 502; 602) inserted into an inside of the catheter body;
aspirating means (11; 111; 511; 611) for aspirating an eliminated thrombus into the inside of the catheter body; and
the atherectomy head (3; 20; 30; 40; 50; 60; 70; 80; 103; 203; 303; 403; 503; 603) according to any one of claims 1 to 5, which is provided on a tip of the atherectomy catheter.

7. An atherectomy catheter according to claim 6, further comprising a visor-like member provided on an opening of the tip of the atherectomy catheter.

8. An atherectomy catheter, comprising:
a freely rotatable hollow cylindrical wire;
rotating means for rotating the cylindrical wire, the rotating means being provided on a rear end thereof;
aspirating means for aspirating an eliminated thrombus into an inside of the cylindrical wire, the aspirating means being provided on a rear end thereof; and
the atherectomy head according to any one of claims 1 to 5, which is provided on a tip of the cylindrical wire.

9. An atherectomy catheter, comprising:
a freely rotatable cylindrical catheter body made of plastic containing a fiber;
rotating means for rotating the catheter body, the rotating means being provided on a rear end thereof;
aspirating means for aspirating an eliminated thrombus into an inside of the catheter body, the aspirating means being provided on a rear end thereof; and
the atherectomy head according to any one of claims 1 to 5, which is provided on a tip of the catheter body.

10. An atherectomy catheter, comprising:
a freely rotatable cylindrical catheter body containing made of plastic containing a mesh;
rotating means for rotating the catheter body, the rotating means being provided on a rear end thereof;
aspirating means for aspirating an eliminated thrombus into an inside of the catheter body, the aspirating means being provided on a rear end thereof; and
the head member according to any one of claims 1 to 5, the the head member being provided on a tip of the catheter body.

## Patentansprüche

1. Atherektomiekopf, umfassend:
eine Klinge (4; 21; 31; 41; 51; 61; 71; 81; 91) zum Entfernen eines Thrombus;
ein Klingenelement (5; 24; 34; 44; 54; 63; 74; 84; 94), welches die Klinge aufweist; und
ein elastisches Element (6; 22; 32; 42; 52; 65; 72; 82; 92), welches aus einem Material ausgebildet ist, welches durch ein Zusammenstoßen mit einem Thrombus verformt wird, wobei das elastische Element eine äußere Fläche aufweist, welche eine Fläche von im Wesentlichen der gleichen Höhe an dem Kopf wie eine Fläche der Klinge umfasst; und **dadurch gekennzeichnet, dass** die Fläche nach außen über die Fläche der Klinge hervorragt, so dass die Spitzenfläche des elastischen Elements durch den Zusammenstoß mit dem Thrombus nach hinten von der Fläche der Klinge eingebeult wird und die Klinge des Klingenelements an den Thrombus stößt, welcher in einer ausgebildeten Beule vorliegt, um den Thrombus zu entfernen.

2. Atherektomiekopf nach Anspruch 1, wobei eine Vertiefung (7; 25; 35; 45; 55; 85; 95) zum Absaugen des entfernten Thrombus in eine Innenseite eines Katheters (1; 101; 201; 301; 401) ausgebildet ist.

3. Atherektomiekopf nach Anspruch 1 oder 2, wobei:
die äußere Fläche des elastischen Elements (22; 32; 42; 72) mit einem Beschichtungselement (23; 33; 43; 73), welches aus einem Material gefertigt ist, welches durch den Zusammenstoß mit dem Thrombus nicht verformt wird, beschichtet ist; und
die Klinge (21; 31; 41; 71) in Berührung mit dem Beschichtungselement ist.

4. Atherektomiekopf nach einem der Ansprüche 1-3, wobei mindestens zwei Klingen (61; 71) als die Klinge bereitgestellt sind.

5. Atherektomiekopf nach einem der Ansprüche 1-4, wobei das elastische Element ein federndes sprungbrettähnliches elastisches Element (82; 92) umfasst.

6. Atherektomiekatheter, umfassend:
einen zylindrischen Katheterkörper (1; 101; 201; 301; 401);
einen frei drehbaren Draht (2; 102; 202; 302; 402; 502; 602), welcher in einer Innenseite des Katheterkörpers eingesetzt ist;
Absaugmittel (11; 111; 511; 611) zum Absaugen eines entfernten Thrombus in die Innenseite des Katheterkörpers; und
den Atherektomiekopf (3; 20; 30; 40; 50; 60; 70; 80; 103; 203; 303; 403; 503; 603) nach einem der Ansprüche 1-5, welcher an einer Spitze des Atherektomiekatheters vorgesehen ist.

7. Atherektomiekatheter nach Anspruch 6, ferner umfassend ein schirmähnliches Element, welches an einer Öffnung der Spitze des Atherektomiekatheters vorgesehen ist.

8. Atherektomiekatheter, umfassend:
einen frei drehbaren hohlen zylindrischen Draht;
Drehmittel zum Drehen des zylindrischen Drahts, wobei die Drehmittel an einem hinteren Ende davon vorgesehen sind;
Absaugmittel zum Absaugen eines entfernten Thrombus in die Innenseite des zylindrischen Drahtes, wobei die Absaugmittel an einem hinteren Ende davon vorgesehen sind; und
den Atherektomiekopf nach einem der Ansprüche 1-5, welcher an einer Spitze des zylindrischen Drahts vorgesehen ist.

9. Atherektomiekatheter, umfassend:
einen frei drehbaren zylindrischen Katheterkörper, welcher aus einer kunststoffenthaltenden Faser gefertigt ist;
Drehmittel zum Drehen des Katheterkörpers, wobei die Drehmittel an einem hinteren Ende davon vorgesehen sind;
Absaugmittel zum Absaugen eines entfernten Thrombus in die Innenseite des Katheterkörpers, wobei die Absaugmittel an einem hinteren Ende davon vorgesehen sind; und
den Atherektomiekopf nach einem der Ansprüche 1-5, welcher an einer Spitze des Katheterkörpers vorgesehen ist.

10. Atherektomiekatheter, umfassend:
einen frei drehbaren zylindrischen Katheterkörper, welcher aus Kunststoff gefertigt ist, welches ein Netz enthält;
Drehmittel zum Drehen des Katheterkörpers, wobei die Drehmittel an einem hinteren Ende davon vorgesehen sind;
Absaugmittel zum Absaugen eines entfernten Thrombus in die Innenseite des Katheterkörpers, wobei die Absaugmittel an einem hinteren Ende davon vorgesehen sind; und
das Kopfteil nach einem der Ansprüche 1-5, wobei das Kopfteil an einer Spitze des Katheterkörpers vorgesehen ist.

## Revendications

1. Tête d'athérectomie, comprenant :
une lame (4 ; 21 ; 31 ; 41 ; 51 ; 61 ; 71 ; 81 ; 91) pour éliminer un thrombus ;
un élément de lame (5 ; 24 ; 34 ; 44 ; 54 ; 63 ; 74 ; 84 ; 94) comprenant la lame ; et
un élément élastique (6 ; 22 ; 32 ; 42 ; 52 ; 65 ; 72 ; 82 ; 92) formé d'un matériau qui est déformé par une collision avec un thrombus, l'élément élastique comprenant une surface extérieure qui comprend une surface à sensiblement la même hauteur sur la tête qu'une surface de la lame ;
et **caractérisée en ce que** ladite surface fait saillie vers l'extérieur au-delà de la surface de la lame,
de sorte que la surface de pointe de l'élément élastique est dentée vers l'arrière à partir de la surface de la lame par la collision avec le thrombus, et la lame de l'élément de lame touche le thrombus présent dans une dent formée pour éliminer le thrombus.

2. Tête d'athérectomie selon la revendication 1, dans laquelle une gorge (7 ; 25 ; 35 ; 45 ; 55 ; 85 ; 95) pour aspirer le thrombus éliminé à l'intérieur d'un cathéter (1 ; 101 ; 201 ; 301 ; 401) est formée.

3. Tête d'athérectomie selon la revendication 1 ou 2, dans laquelle :
la surface extérieure de l'élément élastique (22 ; 32 ; 42 ; 72) est revêtue d'un élément de revêtement (23 ; 33 ; 43 ; 73) fait d'un matériau qui n'est pas déformé par la collision avec le thrombus ; et
la lame (21 ; 31 ; 41 ; 71) est en contact avec l'élément de revêtement.

4. Tête d'athérectomie selon l'une quelconque des revendications 1 à 3, dans laquelle au moins deux lames (61 ; 71) sont fournies chacune en tant que la lame.

5. Tête d'athérectomie selon l'une quelconque des revendications 1 à 4, dans laquelle l'élément élastique comprend un élément élastique résilient semblable à un tremplin (82 ; 92).

6. Cathéter d'athérectomie, comprenant :
un corps de cathéter cylindrique (1 ; 101 ; 201 ; 301 ; 401) ;
un fil métallique pouvant librement tourner (2 ; 102 ; 202 ; 302 ; 402 ; 502 ; 602) inséré à l'intérieur du corps de cathéter ;
des moyens d'aspiration (11 ; 111 ; 511 ; 611) pour aspirer un thrombus éliminé à l'intérieur du corps de cathéter ; et
la tête d'athérectomie (3 ; 20 ; 30 ; 40 ; 50 ; 60 ; 70 ; 80 ; 103 ; 203 ; 303 ; 403 ; 503 ; 603) selon l'une quelconque des revendications 1 à 5, qui est fournie sur une pointe du cathéter d'athérectomie.

7. Cathéter d'athérectomie selon la revendication 6, comprenant en outre un élément semblable à une visière fourni sur une ouverture de la pointe du cathéter d'athérectomie.

8. Cathéter d'athérectomie, comprenant :
un fil métallique cylindrique creux pouvant tourner librement ;
des moyens de rotation pour faire tourner le fil métallique cylindrique, les moyens de rotation étant fournis sur une extrémité arrière de celui-ci ;
des moyens d'aspiration pour aspirer un thrombus éliminé à l'intérieur du fil métallique cylindrique, les moyens d'aspiration étant fournis sur une extrémité arrière de celui-ci ; et
la tête d'athérectomie selon l'une quelconque des revendications 1 à 5, qui est fournie sur une pointe du fil métallique cylindrique.

9. Cathéter d'athérectomie, comprenant :
un corps de cathéter cylindrique pouvant tourner librement fait d'un plastique contenant une fibre ;
des moyens de rotation pour faire tourner le corps de cathéter, les moyens de rotation étant fournis sur une extrémité arrière de celui-ci ;
des moyens d'aspiration pour aspirer un thrombus éliminé à l'intérieur du corps de cathéter, les moyens d'aspiration étant fournis sur une extrémité arrière de celui-ci ; et
la tête d'athérectomie selon l'une quelconque des revendications 1 à 5, qui est fournie sur une pointe du corps de cathéter.

10. Cathéter d'athérectomie comprenant :
un corps de cathéter cylindrique pouvant tourner librement fait d'un plastique contenant un maillage ;
des moyens de rotation pour faire tourner le corps de cathéter, les moyens de rotation étant fournis sur une extrémité arrière de celui-ci ;
des moyens d'aspiration pour aspirer un thrombus éliminé à l'intérieur du corps de cathéter, les moyens d'aspiration étant fournis sur une extrémité arrière de celui-ci ; et
l'élément de tête selon l'une quelconque des revendications 1 à 5, l'élément de tête étant fourni sur une pointe du corps de cathéter.
